# EUROPEAN PATENT APPLICATION

(11) **EP 4 285 877 A1**
(43) Date of publication of application: **06.12.2023**
(21) Application number: 22386035.4
(22) Date of filing: 03.06.2022
(51) Int. Cl.: A61F 9/00, A61F 9/007, A61K 9/00, A61K 31/5575, A61K 47/34, A61P 27/06

(54) **ALTERNATIVE SMART OCULAR IMPLANTS WITH CONTROLLED OPHTHALMIC PHARMACOKINETICS**

(30) Priority: 02.06.2022 GR 20220100465
(71) Applicant: ASLANIS ASLANIDIS, Ioannis, 71201 Heraklion, Crete (GR); Stratakis, Emmanouil, 70013 Heraklion, Crete (GR); Kymakis, Emmanouil, 71410 Heraklion, Crete (GR)
(72) Inventor: ASLANIS ASLANIDIS, Ioannis, 71201 Heraklion, Crete (GR); Stratakis, Emmanouil, 70013 Heraklion, Crete (GR); Kymakis, Emmanouil, 71410 Heraklion, Crete (GR)

(57) **Abstract**

In the proposed embodiment of the invention, is an ophthalmic patch resembling a contact lens, from which the central area is missing, comprising of various systems and devices that provide the following advantages:
i) they are flexible and stable systems for adequate placement on the cornea
ii) they have the appropriate properties (optical, mechanical, hydrophilic, and biological) for controlled drug delivery
iii) they bear a sensor for measuring and recording the intraocular pressure at regular intervals

The invention addresses the disadvantages of the existing glaucoma treatment methods as it is less invasive, since the use of the patch is similar to that of a contact lens, while on the other hand it makes for the first time a combination of specific techniques, materials and technologies for controlled drug release to reduce intraocular pressure. The advantages of the invention include the following: patients' compliance, ease of use, reduction of treatment costs.

## Description

### Technical Field of the invention

### BACKGROUND OF THE INVENTION

The invention relates to the application of eye patch and contact lens technologies. More particularly, the present invention relates to a contact lens-like ophthalmic patch that can act as a controlled drug-release mechanism for the treatment of glaucoma. This invention is based on the use of high-tech lasers for the microstructure of ophthalmic devices in combination with biocompatible graphene structures combined with the on-site integration of an intraocular pressure sensor.

### Prior Technology and its Evaluation

### DESCRIPTION OF RELATED TECHNOLOGY

Glaucoma is treated by reducing intraocular pressure. Depending on the case, the options chosen by the ophthalmologist may include eye drops, oral treatment (pills), laser treatment, surgery or a combination of the above.

Eye drops: Nowadays, the majority of people with glaucoma should use eye drops to treat the problem. Depending on their design, they can reduce the intraocular pressure by improving the drainage of the aqueous humor or reduce the amount of aqueous humor produced by the eye. In some cases though more than one eye drops may need to be prescribed.

The biggest problem with the long-term use of eye drops is that many patients do not adhere to their treatment.

In glaucoma patients, three key factors affect the decision as to whether and how aggressive the treatment will be: a) the patient's survival expectancy, b) the stage of the disease and c) the disease progression rate. In addition, other factors are considered such as the degree of increase of the IntraOcular Pressure (IOP), the presence or absence of bleeding in the optic disc and the family history. The idea of the "target pressure" was introduced as some patients require lower lOPs than others to stabilize their glaucoma. In general, patients who need lower "target pressures" are those with more advanced optic neuropathy. Ophthalmic hypotensive agents are divided into several groups based on their chemical structure and pharmacological action. The groups of agents in widespread clinical use are:
✔ Prostaglandin analogues
✔ B - adrenergic antagonists (non-selective and selective)
✔ Adrenergic agonists
✔ Carbonic anhydrase inhibitors
✔ Parasympathetic mimetic agents (myotics)
✔ Combinations of drugs
✔ Hyperosmotic factors

In this invention any of the above groups of drugs can be used for its controlled release from the ocular patch.

Pills: In case the use of eye drops does not reduce the intraocular pressure to the desired level, the ophthalmologist may also prescribe oral treatment. Possible side effects in this case include frequent urination, tingling in the fingers and toes, depression, stomach upsets and kidney stones.

### Indicative treatments through Laser techniques:

1. Selective Laser Trabeculoplasty (SLT) is performed in order to open the eye's trabeculum. This method has the advantage that it can be repeated, as it does not cause tissue damage, but does not always give the desired result and in many cases is not a permanent solution.
2. In addition, there is the possibility of laser photoagulation (Cyclo-Diode laser) in the radial body of the eye. The aim of this method is to reduce the production rate of the aqueous humor.
3. Moreover, iridotomies can be made around the iris, using a YAG laser so that there is an opening connecting the posterior chamber of the eye with the anterior one.

Surgery: The most common methods of surgery to treat glaucoma include the creation of an artificial aqueous drainage system (trabeculectomy) and the implantation of special valves that drain the fluid the aqueous humor from the eye. Also, in recent years in particular, some other techniques have been developed which are called: Minimally Invasive Glaucoma Surgeries (MIGS). Thanks to these techniques it is possible to use implants, smaller in size than the valves mentioned earlier. These implants are placed in the eye, using specially made devices, through very small incisions and their purpose is to increase the drainage of the aqueous humor in order to reduce the intraocular pressure. As in any surgery, these techniques have the risk of certain problems or complications. Some of these risks include: a) scarring in or on the eyeball, b) eye infection, c) bleeding in the eye, d) draining too much aqueous fluid, leading to very low intraocular pressure, e) cataract, f) loss of vision, g) diplopia, h) possibility of additional corresponding glaucoma surgery or removal of the implant.

Existing related technology: In the international literature and in this technological field, only two applications are more widely known which define the existing level of technology and which have some resemblance to the proposed invention (in that a gradual release of a drug substance is provided). The first one was approved very recently (April 2022)¹ and is entitled Acuvue Theravision with Ketotifen. Acuvue Theravision with Ketotifen is a disposable contact lens that releases the drug ketotifin (antihistamine) to treat the symptoms of allergic conjunctivitis. The drug contained in the lens lasts for up to 12 hours after the application. The second case consists of an implant that was recently developed² The second case consists of an implant that was recently developed and is known by the distinctive title: Durysta. Durysta is a biodegradable implant of prolonged release of the drug called: bimatoprost (prostaglandin analogue), inside the eye which helps to reduce and maintain lower levels of intraocular pressure. This drug is stored in a tiny rod-shaped cartridge which is inserted into the anterior chamber of the eye by an ophthalmologist. In addition to
1 F.D.A approval in 2022, https://www.accessdata.fda.gov/drugsatfda_docs/label/2022/022388s000lbl.pdf
2 F.D.A approval in 2020, https://www.accessdata.fda.gov/drugsatfda_docs/nda/2020/211911Orig1s000TOC.cfm
the potential side effects that a patient may experience from the use of bimatoprost, the use of this implant has been associated with adverse corneal reactions and an increased risk of endothelial cell loss. Therefore, the use of this implant in patients with a limited number of endothelial cells is not recommended. Furthermore, as in all intraocular surgeries and injections, the process of inserting this implant can lead to a serious and often destructive infection inside the eye caused by bacteria or fungi called endophthalmitis.^{3,4}

In all existing technologies and techniques that use implants and valves, the process of their insertion is, to varying degrees, invasive and must be peformed inside the eye, increasing the risk of complications and infections.

### SUMMARY OF THE INVENTION

In the proposed embodiment of the invention, is an ophthalmic patch resembling a contact lens, from which the central area is missing, comprising of various systems and devices that provide the following advantages:
i) they are flexible and stable systems for adequate placement in the corneal area
ii) they have the appropriate properties (optical, mechanical, hydrophilic, and biological) for controlled drug delivery on the cornea
iii) they bear a sensor/system for measuring the intraocular pressure which, according to the dilation-contraction of the eye, will record the intraocular pressure at regular intervals.

The invention addresses the disadvantages of the hitherto known methods. An important difference of the invention from the aforementioned technologies is that on the one hand it is much less invasive, since the use of the patch is similar to that of a common contact lens, while on the other hand it makes for the first time a combination of specific techniques, materials and technologies for controlled drug release to reduce intraocular pressure. The advantages of the invention include the following:
³ https://crstoday.com/articles/supplement/allergan-supplement/
⁴ https://glaucomatoday.com/articles/2021-jan-feb-supplement3/allergan-supplement

Patients' compliance with the medication: The treatment with the use of eye drops that the patient needs is followed normally and the consequences of poor compliance (due to chronic fatigue, other health problems, etc.), which lead to worsening or ineffective treatment of the disease are removed.
Ease of use: With the use of the eye patch of this invention, the stable and correct dosage administration of the medication leading to easy and carefree treatment due to its easy application on the eye.
Reduction of total treatment costs: Due to the full compliance with the eye drops medication achieved thanks to the present invention, all means are exhausted before the use of more invasive methods to reduce intraocular pressure. This reduces the number of patients who will need a form of surgery to implant a valve into the eye that will reduce the pressure inside it.

The invention is defined in the independent claims.

The present invention relates to an ophthalmic implant comprising:
1. An eye patch in the form of a ring, which resembles a contact lens in terms of size and curvature. To enhance the hydrophilicity and adhesion of the patch, its circumference is processed with a pulsed laser. The result of this processing is the creation of a ring with "trench" geometry on the periphery of the implant.
2. Graphene-based binding system for carrying and the controlled release of a drug on the cornea to reduce intraocular pressure.
3. On-site intraocular pressure sensor.

### BRIEF DESCRIPTION OF DRAWINGS

**Figure 1****/****3** illustrates an example of the device according to the invention.
**Figure 2****/****3** shows the process of developing the patch and illustrating the example of the device according to the invention from different angles.
**Figure 3/3** shows the pressure sensor on the eye patch of the invention.

### Disclosure of the Invention

The eye patch is made of materials that are 100% biocompatible, non-toxic and tolerable by the human eye.

The patch consists of one (1) or more rings, peripheral to the pupil of the eye so as not to affect the user's vision.

This ring is formed on the surface of the patch by Ultra-pulse Laser microprocessing (wavelength 250-5000 nm, pulse duration fs-ns, repetition rate 1 Hz-100MHz) to create a hydrophilic contact angle surface of less than 10 degrees. The purpose of this treatment is to create a ring with "trench" geometry on the periphery of the patch. This treatment involves placing transparent materials in a suitable slot; determining the desired geometry of the ring; the determination of the desired focus point and distribution of the laser beam on the surface of the patch; determining the melting point of the patch material; selecting the laser energy density from a range of values; the selection of the wavelength, irradiation rate and duration of the laser pulse from a range of values of wavelength, irradiation rate and pulse duration respectively; selecting the number of continuous radiation pulses per surface of a laser pulse; exposing the surface of the patch to a focused laser beam with a specific wavelength, irradiation rate, pulse duration and number of continuous pulses to increase the surface temperature of the patch material above the melting point to build at least one point on the surface and creating at least part of the desired structure.

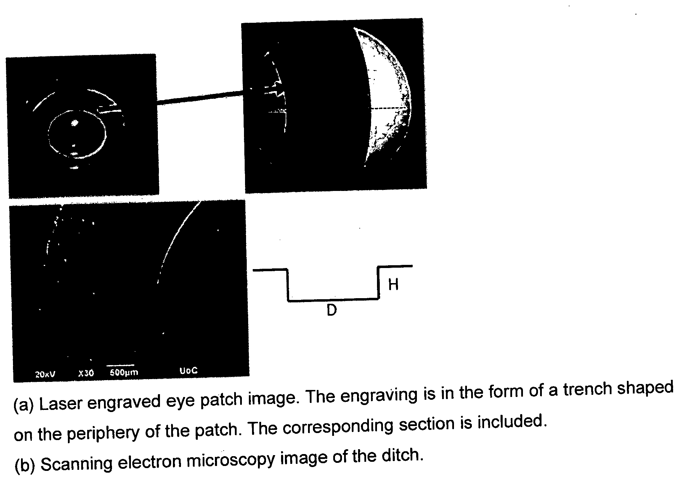

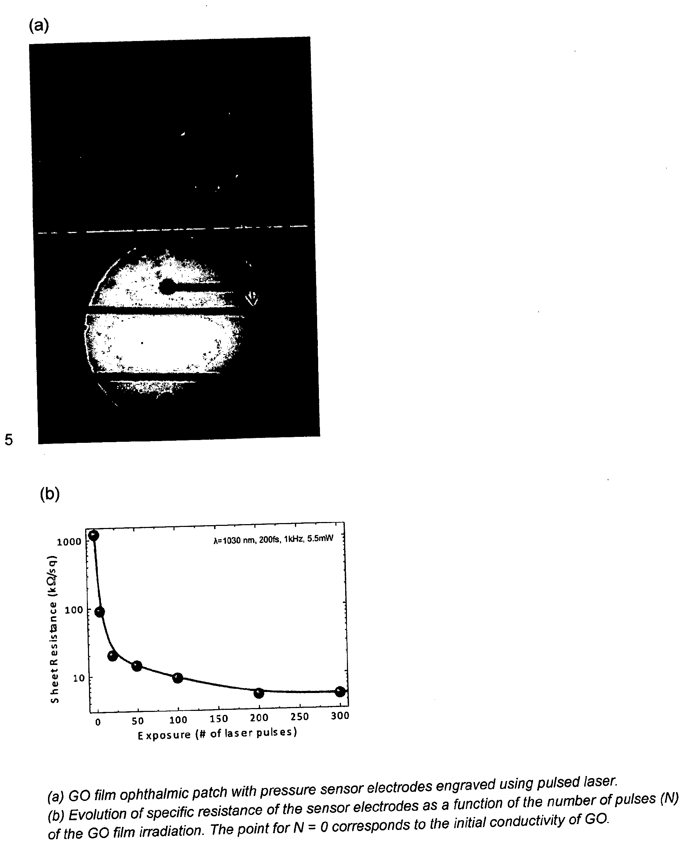

The result of processing the transparent materials with laser pulses is the creation of a trench-shaped ring on the periphery of the patch (Figure 1/3: ring and SEM ring). The creation of this ring has the effect of enhancing the hydrophilicity of the patch.

A ring is developed in this ring to bind, transport and control the medication release to reduce the intraocular pressure. This platform is based on two-dimensional (2D) graphene-based chemical structures. The selected graphene structure for the drug delivery system is prepared by two chemical processes from graphite precursor material. Specifically, starting from graphite, graphite oxide is prepared using a modified Hummers' method, which is a standard chemical method for making graphite oxide (GO). Subsequently, using practices widespread and commonly accepted by the scientific community, graphene oxide is subjected to partial chemical reduction using a non-toxic, biocompatible, mild reducing agent to produce Reduced Graphene Oxide.

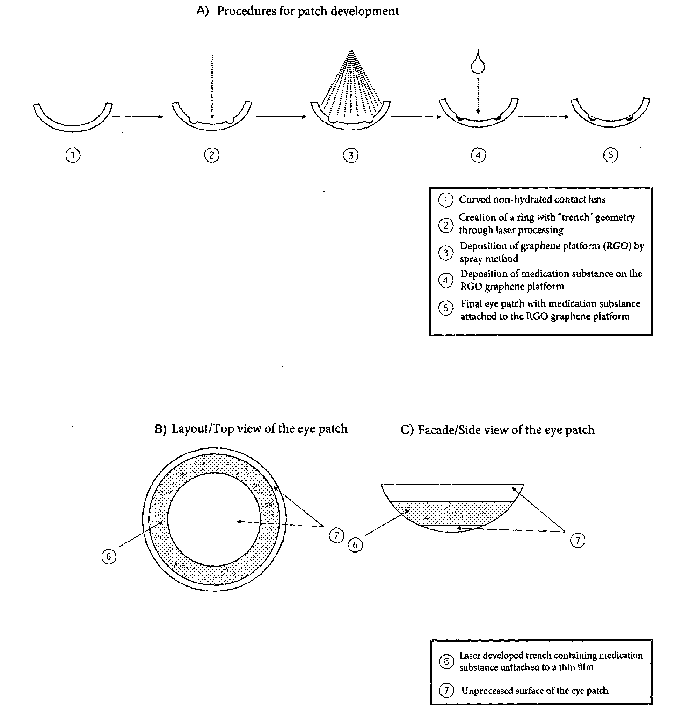

The final RGO suspension is then deposited inside the ophthalmic patch by spraying, using an airbrush and a nitrogen gas (N2) air compressor. After spraying, excess RGO film is removed with high-precision micro cleaning swabs, so that RGO is present only in the trench created by the laser radiation inside the contact lens. The result is the development of a thin solid RGO film to which the antiglaucoma drug binds. (Figures 1/3 and 2/3).

The implementation of this patch will include a sensor for monitoring intraocular pressure.

In one embodiment of this patch the monitoring is done by connecting to an external device while in another embodiment this patch will be performed using a portable device.

The sensor system for monitoring intraocular pressure is based on the engraving of reduced graphene oxide electrodes on the outer side of the ocular patch, which will coincide with a part of the limbus of the eye (Figure 3/3). According to relevant literature ^{5,6,7,8,9} the change in the dilation of the cornea due to the increase of the intraocular pressure causes dilation of the patch. This dilation is more intense and therefore more easily detectable, especially in the area of the limbus⁶ due to the larger radius at this point in relation to points closer to the center of the patch (as is the case with the cornea). This change causes a change in the conductivity of the reduced graphene^{4,5} by 2-5 orders of magnitude (Figure 3/3), which in the proposed embodiment is detected by measuring the resistance of the electrode using a device for electrical characterization using pins.

The reduction of graphene oxide in the sensor implementation is carried out by irradiating the area of interest using a pulsed laser (wavelength 250-5000 nm, pulse duration fs-ns, repetition rate 1Hz-100MHz) in a layer of graphene oxide already
⁵ Liu Z, Wang G, Pei W, Wei C, Wu X, Dou Z, et al. Application of graphene nanowalls in an intraocular pressure sensor. Journal of Materials Chemistry B 2020;8:8794-802.
⁶ Zhang Y, Chen Y, Man T, Huang D, Li X, Zhu H, et al. High resolution non-invasive intraocular pressure monitoring by use of graphene woven fabrics on contact lens. Microsystems & Nanoengineering 2019;5:39.
⁷ Dou Z, Tang J, Liu Z, Sun Q, Wang Y, Li Y, et al. Wearable Contact Lens Sensor for Non-Invasive Continuous Monitoring of Intraocular Pressure. Micromachines 2021;12:108.
⁸ Xu J, Cui T, Hirtz T, Qiao Y, Li X, Zhong F, et al. Highly Transparent and Sensitive Graphene Sensors for Continuous and Non-invasive Intraocular Pressure Monitoring. ACS applied materials & interfaces 2020;12:18375-84.
⁹ Kim J, Kim M, Lee MS, Kim K, Ji S, Kim YT, et al. Wearable smart sensor systems integrated on soft contact lenses for wireless ocular diagnostics. Nature communications 2017;8:14997.
deposited in the outer side of the patch using the spray technique mentioned earlier. This reduction is performed by scanning the surface of the patch with a laser beam in the area of the ring. The high scanning speed of the surface with multiple pulses in a row (eg 3 to 5 pulses per focus area) causes the oxygen groups to be removed from the GO grid. The distance between the scans can be adjusted approximately equal to the diameter of the irradiated surface. This reduction leads to the improvement of the conductivity of the film by 2-3 orders of magnitude (Figure 3/3).

The area of interest in the implementation of this patch is 1 mm - 3 mm along the perimeter zone of the patch.

In the proposed embodiment of the patch, the part of the graphene oxide layer that has not been reduced is removed by placing the patch in an aqueous environment in order to reduce the patch layers and not to interfere with its transparency.

In one embodiment of the patch the material to be used as a base will a hydrogel.

In another embodiment of the patch the material to be used as a base will be silicone. In another embodiment of the patch the material to be used as a base will be a silicone-hydrogel combination.

In an embodiment of the eye patch, based on any of the above materials, it will have a diameter of 13.8 - 14.5mm and will be easily tolerated by the user.

In one embodiment of the eye patch, based on any of the above materials and any of the above diameters, the curvature (curvature that shows how convex the lens must be to be comfortable to the eye and move freely on the cornea to ensure the flow of tears between the cornea and the patch), may be 8.4 or 8.6 or 8.8.

In an embodiment of the eye patch, based on any of the above materials, any of the above diameters and any of the above curvatures, the water content may belong to any of the following hydration categories: low water saturation (up to 45 %) - medium water saturation (45-55%) - high water saturation (over 55%).

In an embodiment of the eye patch, based on any of the above materials, any of the above diameters, any of the above curvatures, any of the above hydration categories, the thickness of the patch as thin as of the order of 0.10 mm.

The main purpose of the eye patch is the controlled release of a drug on the cornea of the eye in order to reduce intraocular pressure. At the same time, with the sensor that is integrated, it is possible to periodically measure the pressure of the eye so that on the one hand there is a complete recording of the pressure fluctuation in a certain period of time while on the other hand the effectiveness of the patch itself to reduce intraocular pressure can be monitored.

An embodiment of the eye patch is illustrated in **Figure 1****/****3** and analyzed below.

In another embodiment of the eye patch the pressure sensor will be, as in **Figure 3/3**.

The advantages of the eye patch of the invention over the traditional methods of reducing intraocular pressure include the following: i) stable and correct drug dosage application to the patient, ii) easy and carefree treatment, iii) easy placement on the eye.

Thanks to the patch of the present invention, the stable and correct release of a medication substance on the cornea is ensured, with a procedure that is not at all invasive and that resembles the use of a contact lens.

The use of the invention is described below by means of an example and with reference to the accompanying drawings.

The patient sits in an examination chair as in a routine ophthalmological examination. The ophthalmologist places the patch (Figure 1 (a)/3) following the same procedure as when applying a contact lens. On the patch itself, there is the platform for binding, transporting and controlled release of the medication substance (Figure 1 (b)/3) as well as an intraocular pressure sensor (Figure 3 (a)/3). The patient can then continue his normal activities until the next visit to his ophthalmologist.

It is obvious that with the use of the eye patch, a reduction of the intraocular pressure is achieved without creating particular problems during its use.

## Claims

1. Pulsed Laser eye patch processing method (wavelength 250-5000 nm, pulse duration fs-ns, repetition rate 1Hz-100MHz) to improve the hydrophilicity of the patch. The treatment concerns the formation of a ring 0.01 - 4.0 mm wide and 0.001-5.0 mm deep, trench-shaped, in the circumference of the patch.

2. Method of depositing the GO film on the outside of the patch by the GO aqueous suspension spray method, using an airbrush and an air compressor under nitrogen gas flow (N₂).

3. Method of processing the GO film on the patch with Pulsed Laser (wavelength 250-5000 nm, pulse duration fs-ns, repetition rate 1Hz-100MHz) to reduce GO to rGO and improve its conductivity by 2-3 orders of magnitude.

4. An eye patch system consisting of one (1) or more (Figure 1 (b)/3) trench-shaped rings, pulsed by laser processing, around the pupil of the eye (Figure 1 (a)/3), comprising:
• biocompatible materials as the basis of the patch itself
• graphene oxide film
• medication to reduce intraocular pressure
• intraocular pressure sensor

5. A system according to claim **1**, wherein the drug for reducing intraocular pressure is replaced by another ophthalmic drug.

6. A system according to claims **1 and 2**, wherein the sensor for monitoring the intraocular pressure will be connected to an external device.

7. System according to claims **1, 2** and **3**, wherein the pressure sensor for intraocular pressure monitoring will be connected to a portable device.

8. Method for monitoring the intraocular pressure using the system of claims **1 to 4**, wherein the method comprises the following steps:
• availability of an eye patch based on biocompatible graphene structures
• availability of a platform for binding, transport and controlled release of medication substance on the cornea of the eye
• availability of a sensor to measure the intraocular pressure

9. A method according to claims **1 and 2**, wherein the method comprises the following steps:
• availability of an eye patch based on biocompatible graphene structures
• availability of a platform for the binding, transport and controlled release of a medication on the cornea of the eye
